# EUROPEAN PATENT SPECIFICATION

(11) **EP 3 245 215 B1**
(45) Date of publication and mention of the grant of the patent: **07.06.2023**
(21) Application number: 16737971.8
(22) Date of filing: 15.01.2016
(51) Int. Cl.: A61K 31/683, A61P 25/00, A61P 25/08

(54) **METHODS OF TREATING NEUROLOGICAL INFLAMMATORY DISORDERS**
VERFAHREN ZUR BEHANDLUNG VON NERVENENTZÜNDUNGSERKRANKUNGEN
MÉTHODES DE TRAITEMENT DE TROUBLES INFLAMMATOIRES NEUROLOGIQUES

(30) Priority: 15.01.2015 US 201562103629 P
(43) Date of publication of application: 22.11.2017
(73) Proprietor: Mayo Foundation for Medical Education and Research, Rochester, MN 55905 (US)
(72) Inventor: HOWE, Charles L., Rochester, Minnesota 55906 (US)
(74) Representative: Fish & Richardson P.C.
(86) International application number: PCT/US2016/013622
(87) International publication number: WO 2016/115473

(56) References cited:
- US-A1- 2008 085 306
- US-A1- 2010 286 082
- US-A1- 2012 245 202
- US-A1- 2014 179 750
- US-A1- 2014 303 367
- Hitzert ET AL: "Favorable Outcome in a Newborn With Molybdenum Cofactor Type A Deficiency Treated With cPMP", , 1 October 2012 (2012-10-01), XP55314000, DOI: 10.1542/peds.2011-3330 Retrieved from the Internet: URL:http://pediatrics.aappublications.org/ content/pediatrics/130/4/e1005.full.pdf [retrieved on 2016-10-26]
- DATABASE WPI Week 201142 Thomson Scientific, London, GB; AN 2011-G66521 XP002783401, & JP 2011 116700 A (GH KAKEI GAKUEN) 16 June 2011 (2011-06-16)
- VELDMAN A ET AL: "EFFICACY AND SAFETY OF CYCLIC PYRANOPTERIN MONOPHOSPHATE IN THE TREATMENT OF SIX NEWBORN PATIENTS WITH MOLYBDENUM COFACTOR DEFICIENCY TYPE A", JOURNAL OF INHERITED METABOLIC DISEASE, vol. 34, no. Suppl. 3, 2011, page S84, XP9507100, & ANNUAL SYMPOSIUM OF THE SOCIETY-FOR-THE-STUDY-OF-INBORN-ERRORS-OF- METABOLISM (SSIEM) / MEETING OF TH; GENEVA, SWITZERLAND; AUGUST 30 -SEPTEMBER 02, 2011

## Description

### CROSS REFERENCE TO RELATED APPLICATIONS

This application claims the benefit of U.S. Provisional Serial No. 62/103,629 filed January 15, 2015.

### TECHNICAL FIELD

The present invention generally relates to the treatment of neurological inflammatory diseases.

### BACKGROUND

Molybdenum cofactor (MoCo) is an evolutionarily conserved molybedenum (Mo) coordinated pterin-compound and is necessary for the activity of all Mo-enzymes, with the exception of nitrogenase. MoCo is synthesized by a unique and evolutionarily conserved multistep pathway, from which only two intermediates have been identified: the sulphur- and metal-free pterin derivative, precursor Z, also known as cPMP, and molybdopterin (MPT), a pterin with an ene-dithiol function, which is essential for the Mo-linkage.

Hitzert et al. describe in Pediatrics, 2012;130:e1005-e1010, a favorable outcome in a newborn with MoCo Type A deficiency treated With cPMP.

Veldman et al. describe in Journal of Inherited Metabolic Disease, vol. 34, no. Suppl. 3, 2011, on page S84 the efficacy and safety of cPMP in the treatment of six newborn patients with MoCo Type A deficiency.

### SUMMARY

The present invention is defined by independent claim 1. The dependent claims depict additional embodiments of the invention. Any subject-matter falling outside the scope of the claims is provided for information purposes only.

Compositions for use in methods of treating neurological inflammatory disease or seizures caused by neuroinflammation by administering cPMP to patients having ALS, epilepsy, autism or schizophrenia are described. Treating neuroinflammatory and neurometabolic diseases with cPMP is described so as to override dyshomeostasis in the MoCo synthesis pathway and control synaptic inhibition in the gephyrin-GABAR pathway. This is a novel strategy for preventing neural circuit dyshomeostasis by stabilizing inhibitory synapses.

In one aspect, a composition for use in a method of treating a neurological inflammatory disease in an individual having ALS, epilepsy, autism or schizophrenia is provided. The method includes administering an effective amount of cPMP to the individual, thereby treating the individual. The method further includes identifying an individual having amyotrophic lateral sclerosis (ALS), epilepsy, autism, or schizophrenia. Representative neurological inflammatory diseases include, without limitation, central nervous system (CNS) autoimmune disorders such as autoimmune epilepsies; ALS; schizophrenia; autism; epilepsy and febrile seizures without underlying infection.

In some embodiments, the administering step is selected from the group consisting of orally administering, topically administering, and parenterally administering. In some embodiments, the method further includes identifying an individual having a mutation in a gene selected from the group consisting of gephyrin, MOCS 1, and MOCS2. In some embodiments, the method further includes monitoring the individual for the amount of MPT, MoCo, MoCo-S or another intermediate or by-product of the MoCo biosynthesis pathway. The references to methods of treatment in the detailed description of the invention in this description are to be interpreted as references to the compounds, pharmaceutical compositions and medicaments of the present invention for use in a method for treatment of the human (or animal) body by therapy.

Unless otherwise defined, all technical and scientific terms used herein have the same meaning as commonly understood by one of ordinary skill in the art to which the methods and compositions of matter belong. In addition, the present materials, uses and examples are illustrative only.

### DESCRIPTION OF DRAWINGS

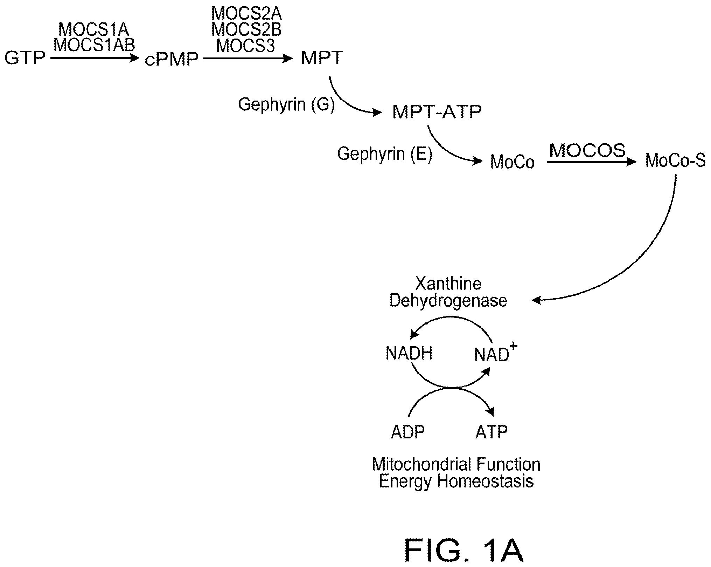

Figure 1A is a schematic showing the synthesis of molybdenum co-factor under homeostatic conditions.

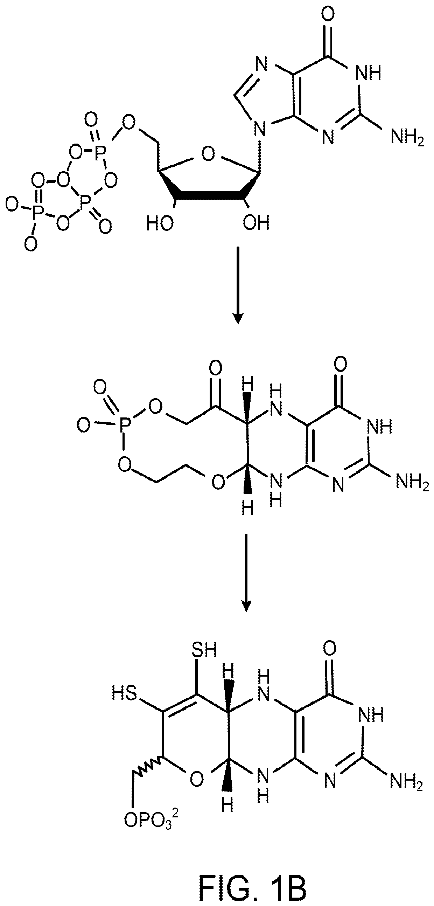

Figure 1B are the chemical structures of the first three compounds shown in Figure 1A: guanosine triphosphate (GTP), cyclic pyranopterin monophosphate (cPMP), and molybdopterin (MPT).

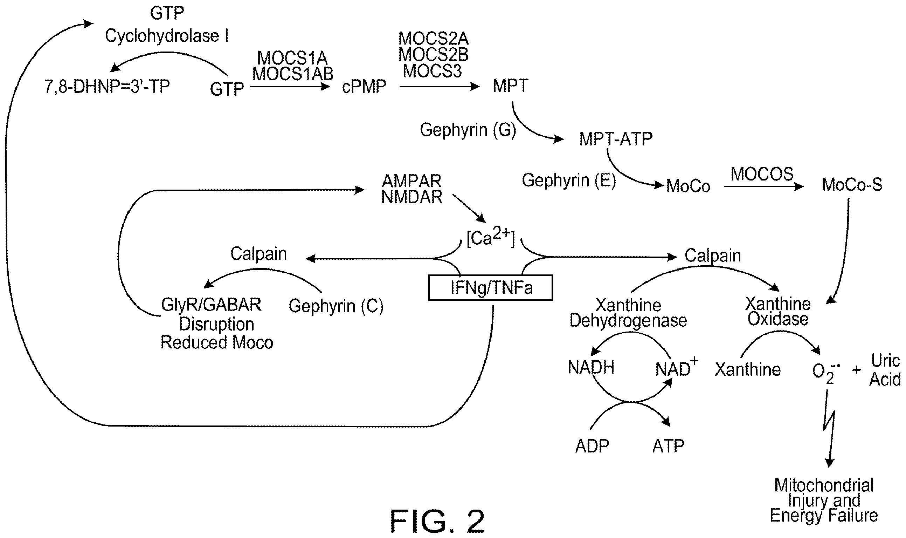

Figure 2 is a schematic showing the synthesis of molybdenum co-factor during inflammatory conditions.

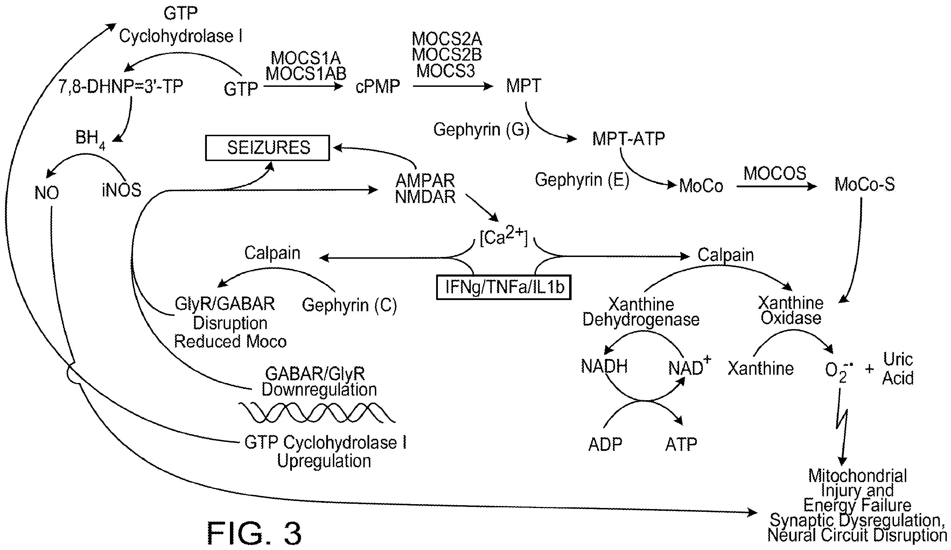

Figure 3 is a schematic showing a summary of the impact of inflammatory cytokines on elements of the molybdenum biosynthesis pathway and the concomitant dysregulation of inhibitory synaptic function that results in hyperexcitability and seizure.

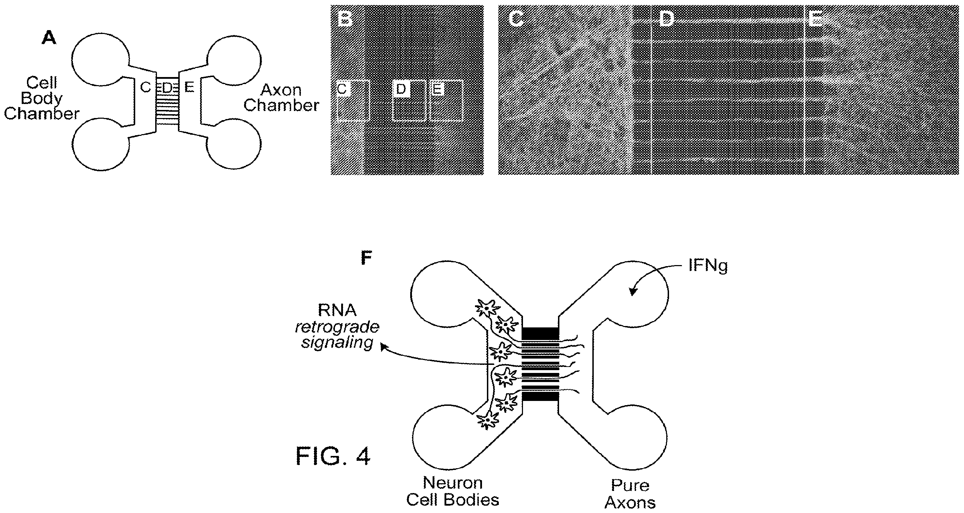

Figure 4 demonstrates the IFNgamma-induced dysregulation of the MoCo pathway and down-regulation of inhibitory synaptic proteins. Mouse cortical neurons were cultured in a two-chamber device that separates cell bodies from axons. Panel A is a schematic of the chambered device constructed in PDMS polymer. Panel B is a low-magnification image of the corresponding regions shown in Panel A stained with an antibody against neurofilament (an axon-specific protein). Higher magnification images are shown of the cell body chamber (Panel C), the axon grooves (Panel D), and the axon chamber (Panel E). DAPI staining indicates the complete absence of any cells in the axon chamber. Panel F is a schematic showing the experimental design: IFNgamma was added to the pure axons in the axon chamber; RNA was collected from the cell body chamber 72 hours later and analyzed by microarray to identify changes in gene expression.

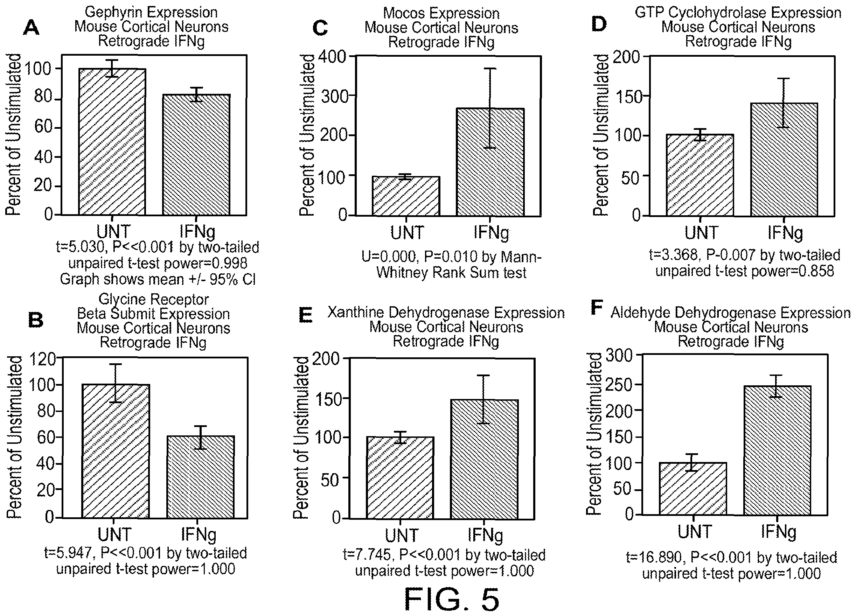

Figure 5 are graphs showing that IFNgamma stimulation of the distal axons stimulated a transcriptional program in the neuron cell bodies that is marked by simultaneous down-regulation of numerous components of inhibitory synapses (gephyrin (Panel A), glycine receptor beta subunit (Panel B), numerous GABA receptor elements (not shown), and multiple gephyrin-binding scaffolds (not shown)) and robust up-regulation of MOCOS (Panel C). At the same time, GTP cyclohydrolase I (Panel D), xanthine dehydrogenase (Panel E), and aldehyde dehydrogenase (Panel F) were significantly up-regulated, indicating substantial changes in the MoCo pathway.

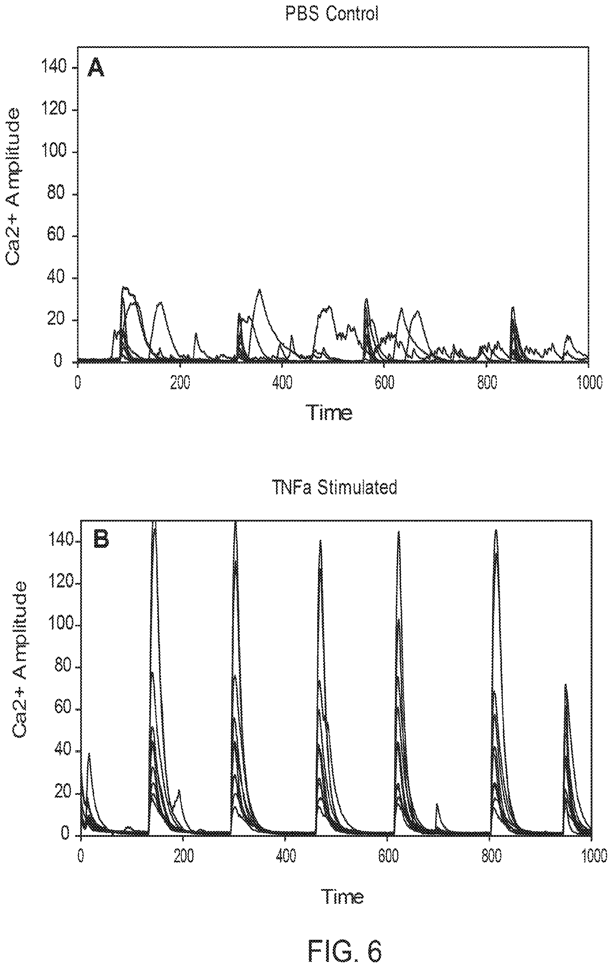

Figure 6 shows spontaneous calcium levels in neurons stimulated for 24 hr with TNFalpha or unstimulated (PBS, vehicle control). The PBS control cultures show low amplitude calcium transients that are non-synchronous. In contrast, stimulation with TNFalpha drove the cells to exhibit large amplitude calcium signals that were highly synchronized, indicating a general reduction in synaptic inhibition in the neural network. Calcium levels were monitored using a fluorescent reporter transduced into the neurons with adenovirus.

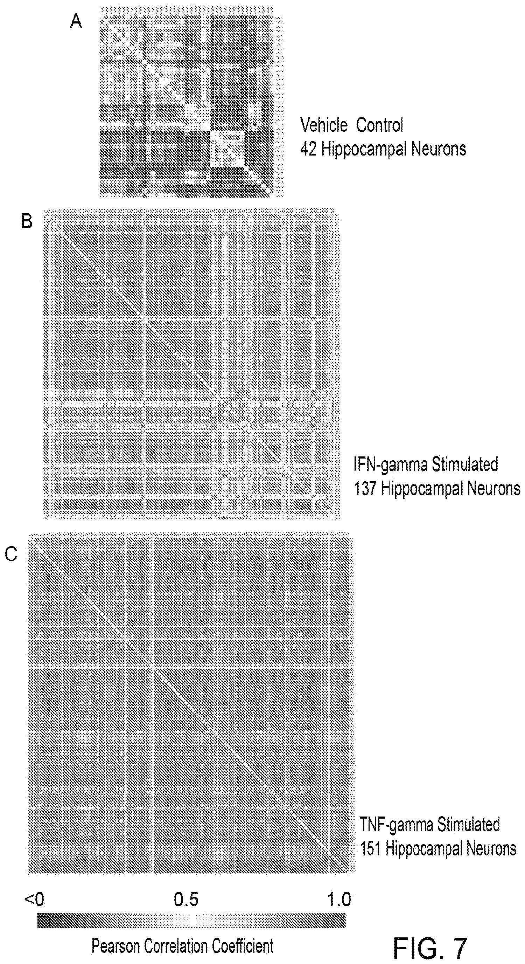

Figure 7 demonstrates that spontaneous activity in neurons stimulated with TNFalpha or IFNgamma is highly correlated (hence, synchronous), in contrast to vehicle control-stimulated cultures. The Pearson correlation matrix was calculated for all of the neurons showing spontaneous activity in the culture and heat-mapped (red = highly correlated activity; blue = uncorrelated activity). In the vehicle control neurons, there is a small cluster of synchronous activity but the overall network is uncorrelated. TNFalpha or IFNgamma stimulation resulted in nearly complete correlation across the entire network. Moreover, the absolute number of spontaneously active cells is clearly increased in the IFNgamma and TNFalpha stimulated networks (137 neurons and 151 neurons, respectively, versus only 42 neurons in the vehicle control). These findings indicate that inhibition in the network is highly suppressed by TNFalpha or IFNgamma stimulation.

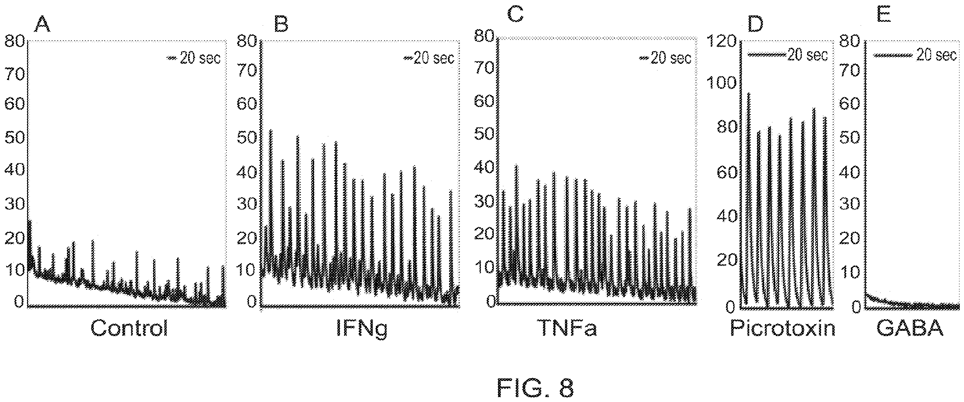

Figure 8 are graphs showing the averaged calcium responses in cytokine-stimulated neurons. Figure 8A shows the basal levels of activity in the neuron cultures. Non-synchronized calcium responses occur in the control cultures, resulting in an overall low level of synaptic activity in the network. Figures 8B and 8C show that stimulation with IFN gamma or TNF alpha results in network bursting and highly synchronized synaptic activity in which many cells in the culture flux calcium at the same time. Figure 8D shows that treatment of control cultures with picrotoxin (2.4 µM), a small molecule inhibitor of inhibitory GABAergic channels, induces network synchrony and bursting that phenocopies the response observed in cytokine-stimulated cultures. Figure 8E shows that the addition of GABA (27 µM) to control cultures completely suppresses synaptic activity, consistent with enhanced inhibition.

### DETAILED DESCRIPTION

As described herein, modulation of the molybdenum cofactor biosynthetic pathway returns cells that are under inflammatory stress to homeostasis by reducing shunting of gephyrin away from stabilization of inhibitory synapses and renormalizing inhibitory control of neural networks. Therefore, as described herein, cPMP can be administered to an individual to treat a number of different neurological inflammatory diseases or relieve the symptoms that are a result of a number of different neurological disorders.

### Molybdenum Cofactor (MoCo) and the Genetics Associated Therewith

All of the molybdenum (Mo) containing enzymes of humans, animals, plants, arachaea and bacteria, with the exception of nitrogenase from prokaryotes, require a co-factor that includes an organic moiety, molybdopterin (MPT), and molybdenum. This molybdenum cofactor (MoCo) possesses, across all phylogenetic groups, the same base structure that is very unstable in its free form, in particular under aerobic conditions when it is not bound to an apoprotein. The biosynthetic pathway, discussed in more detail below, is evolutionarily conserved and the corresponding proteins from various organisms are extremely homologous.

A mutational defect in MoCo-biosynthesis leads to simultaneous loss of the activities of all Mo enzymes, inclusive the sulphite oxidase. Human MoCo deficiency is a severe, autosomal-recessive genetic disorder, which clinically cannot be differentiated from the less frequently occurring sulphite-oxidase deficiency. Most afflicted patients exhibit neurological abnormalities such as non-treatable seizures and lack of development of the brain, which can be traced back to the toxicity of sulphite, a lack of sulphate or both. Most afflicted patients usually die in early childhood.

A eukaryotic gene encoding a protein involved in MoCo-biosynthesis was obtained from *Arabidopsis thaliana.* Subsequently, a human gene encoding a protein involved in MoCo-biosynthesis, was obtained and designated MOCS1. Due to alternate splicing of the MOCS1 gene, the MOCS1A and MOCS1B proteins are produced and convert a guanosine derivative into the sulphur-free precursor Z (i.e., cPMP). Patients having a mutation in the MOCS1 gene are referred to as having MoCo-deficiency type A. In a subsequent step, precursor Z (i.e., cPMP) is converted to MPT by an enzyme, which is encoded by a gene designated MOCS2 and activated by the protein encoded by the MOCS3 gene. Patients having a mutation in the MOCS2 gene are referred to as having MoCo-deficiency type B. Finally, Mo is inserted into MPT by a protein referred to as gephyrin. Patients having a mutation in the gene encoding gephyrin, GEPHN, are referred to as having MoCo-deficiency type C.

### Inflammation-Induced Injury and the MoCo Biosynthesis Pathway

Inflammation triggers neuronal and axonal injury via multiple mechanisms. However, perhaps the most physiologically relevant mechanism of neuronal injury is inflammation-induced synaptic dysfunction and derailment of homeostatic electrophysiological activity in neural circuits. For example, TNFalpha and IFNgamma are known to induce hippocampal injury by triggering excitotoxicity. There are multiple mechanism by which these inflammatory cytokines alter synaptic function - for example, by altering excitatory receptor function and increasing synaptic calcium levels. However, an equally important mechanism of cytokine-mediated synaptic dysregulation may be down-regulation of inhibitory receptors. Reduced inhibition will raise the overall level of synaptic activity and create a feedback loop in which excitatory synaptic activity builds, calcium accumulates in the synapse, and calcium-dependent proteases degrade synaptic connections. This feedback loop likely exacerbates the loss of inhibition, creating spreading synaptic dysregulation, neural injury, and neural circuit hyperactivity and/or failure.

Gephyrin is a critical scaffolding protein that controls the localization, clustering, and inhibitory function of glycine and GABA receptors at synaptic sites. Gephyrin function is directly tied to inhibitory control of neural circuitry, and down-regulation of gephyrin is linked to seizures and hyperexcitability of neurons. Genetic defects in gephyrin are associated with autism, epilepsy, and schizophrenia.

The inhibitory receptor scaffolding function of gephyrin is mediated by a C domain that links evolutionarily conserved G and E domains. Crucially, the G and E domains of gephyrin are necessary for the synthesis of molybdenum cofactor (MoCo), a molecule that is required for activation of molybdenum-dependent enzymes necessary for survival. Humans with mutations in the non-scaffolding domains of gephyrin exhibit MoCo deficiencies and severe neurological and developmental abnormalities.

Under homeostatic conditions, guanosine triphosphate (GTP) is converted to a coordination complex of molybdopterin and a molybdenum oxide (MoCo) by the action of several catalytic enzymes including gephyrin. MoCo must be sulfurated by the molybdenum cofactor sulfurase (MOCOS) in order to function as a co-factor for xanthine dehydrogenase and other molybdenum-dependent enzymes. Xanthine dehydrogenase catalyzes the conversion of xanthine and NAD+ to urate and NADH, providing a fundamental reducing agent necessary for redox metabolism and the production of cellular energy stores in the form of ATP.

Increases in cellular calcium lead to the activation of calcium-dependent proteases such as calpain. Calpain targets two components of the MoCo biosynthesis pathway, resulting in disruption of cellular homeostasis. Calpain irreversibly converts xanthine dehydrogenase to xanthine oxidase, creating a powerful source of reactive oxygen species that directly damage the cell. Moreover, the conversion of xanthine dehydrogenase to xanthine oxidase shunts cellular metabolism away from the production of NADH and ATP, compromising cellular energy balance. Calpain also cleaves gephyrin, resulting in loss of scaffolding function and down-regulation of inhibitory synaptic function. Calpain-cleaved gephyrin also exhibits altered MoCo synthesis function caused by the physical separation of the G and E domains.

Calpain-mediated cleavage of gephyrin at synapses creates a feedback loop in which reduced inhibitory receptor function results in increased excitatory receptor activity, increased calcium influx, and further activation of calpain.

Inflammatory cytokines such as IFNgamma and TNFalpha directly alter calcium flux in target cells and increase expression and activation of calpain. Inflammatory cytokine exposure will therefore reduce inhibitory synaptic function, increase excitatory load, alter MoCo synthesis, and drive the target cell toward reactive oxygen species production.

Inflammatory cytokines also increase the expression of GTP cyclohydrolase I, the rate limiting step in the de novo synthesis of 5,6,7,8-tetrahydrobiopterin from GTP. Inflammatory cytokine exposure will therefore shunt GTP away from MOCS1A/MOCS1AB-mediated production of cyclic pyranopterin monophosphate (cPMP), resulting in decreased MoCo synthesis.

### Seizures and the MoCo Biosynthesis Pathway

Febrile seizures are the most common type of neurologic complication in infants and preschool children. Febrile seizures occur at body temperatures over 38°C in the absence of acute electrolyte imbalance or dehydration, in the absence of direct CNS infection, and without previous evidence of unprovoked seizures (Commission on Epidemiology and Prognosis, 1993, Epilepsia, 34:592-6). It is estimated that 1 in 25 children will experience at least one febrile seizure, and the occurrence of febrile seizure is associated with heightened susceptibility to future seizures - 1 in 3 individuals with childhood febrile seizure will experience another seizure of some type within 20 years. Moreover, the risk of epilepsy among individuals experiencing a childhood febrile seizure is higher than the general population, with incidence reports ranging from 6% to 13%, rates that are more than 10 times higher than in the general population. Of note, an increased frequency of febrile seizure is associated with some vaccines in children, including measles-containing and pertussis vaccines. For example, the diptheria-tetanus-pertussis vaccine is associated with an increase of 6-9 cases of febrile seizure per 100,000 vaccinations and fever is observed in 50% of vaccinated infants. The measles-mumps-rubella (MMR) vaccine is associated with an increase of up to 16 cases of febrile seizure per 100,000 vaccinations, and the addition of varicella to the same vaccine increases the risk even further. Finally, acute seizures associated with viral, bacterial, and parasitic infections in children, whether systemic or localized to the CNS, are a primary factor in the development of epilepsy. For example, during the 2009-2010 influenza A (H1N1) pandemic, in which more than 70% of infected individuals were younger than 24 years of age, up to 6% of infections resulted in neurological complications, with over 10% of children less than 15 years of age presenting with neurological symptoms. Of these complications, seizure and abnormal EEG were the most common. Likewise, infection with enterovirus 71, a picornavirus with widespread epidemic infectivity throughout the Asia-Pacific region, is associated with neurologic complications in almost 20% of infected children. A high incidence of seizure also occurs in children infected with *Plasmodium, Taenia solium* and other parasites. The common factor across all of these seizure events, whether febrile or afebrile, is the production of inflammatory cytokines in the CNS. Interleukin-1beta (IL-1beta) and tumor necrosis factor alpha (TNFalpha) are powerful pyrogens that are elevated in the brain during febrile seizures, and experimental evidence directly supports a role for these factors in the initiation of seizures. Likewise, TNFalpha, interleukin-6 (IL-6), and interferon gamma (IFNgamma) are produced and/or released in the CNS during acute infection.

### Treating Neurological Inflammatory Diseases

As described herein, bypassing GTP-to-cPMP conversion by providing exogenous cPMP can stabilize MoCo synthesis and provide regulatory feedback control to drive a dysregulated system back toward homeostasis. Similarly, blocking GTP cyclohydrolase I to push GTP back into the MoCo pathway; increasing expression or activity of MOCS1A and/or MOCS1AB activity to push GTP to cPMP; or increasing expression or activity of MOCS2A, MOCS2B, and/or MOCS3 activity to push cPMP to MPT also can stabilize MoCo synthesis and provide regulatory feedback control to drive a dysregulated system back toward homeostasis. Likewise, increasing expression or activity of gephyrin to increase MoCo synthesis and to stabilize inhibitory synapses; or blocking calpain to prevent the conversion of xanthine dehydrogenase to xanthine oxidase can stabilize MoCo synthesis and provide regulatory feedback control to drive a dysregulated system back toward homeostasis. Supplementation with cPMP may be enhanced by simultaneously blocking calpain to prevent aberrant xanthine oxidase-dependent production of reactive oxygen species during an inflammatory drive and to maintain gephyrin-dependent synaptic stabilization. In addition, given the link between inflammation-induced seizures and gephyrin/GABAR, treatment with glycogen synthase kinase 3beta (GSK3beta) inhibitors may increase gephyrin activity. For example, GSK-3 Inhibitor IX (CAS 667463-62-9) or lithium chloride may suppress seizures by enhancing gephyrin function and overcome inflammation-induced shunting of gephyrin away from inhibitory synapse stabilization. Thus, methods of treating a neurological inflammatory disease are described herein.

As used herein, neurological inflammatory diseases include, without limitation, central nervous system (CNS) autoimmune disorders such as multiple sclerosis (MS), neuromyelitis optica (NMO), anti-NMDA receptor encephalitis, and autoimmune epilepsies; Alzheimer's disease; amyotrophic lateral sclerosis (ALS); schizophrenia; autism; epilepsy and other seizure disorders (e.g., febrile seizures without underlying infection); CNS infectious diseases (e.g., viral, bacterial, parasitic); MoCo deficiencies (e.g., due to genetic mutations); and other neurodegenerative diseases involving microglial and astrocytic inflammatory responses. A neurological inflammatory disease for which the methods described herein are particularly useful is neuroinflammation-induced seizures. "Treating" as used herein refers to relieving, reducing or ameliorating the symptoms of any of such neurological inflammatory diseases.

As described herein, treating a neurological inflammatory disease can include administering an effective amount of cPMP to an individual. In some instances, an individual may be identified as having a neurological inflammatory disease (e.g., central nervous system (CNS) autoimmune disorders such as multiple sclerosis (MS), neuromyelitis optica (NMO), anti-NMDA receptor encephalitis, and autoimmune epilepsies; Alzheimer's disease; amyotrophic lateral sclerosis (ALS); schizophrenia; autism; epilepsy and other seizure disorders (e.g., febrile seizures without underlying infection); CNS infectious diseases (e.g., viral, bacterial, parasitic); MoCo deficiencies (e.g., due to genetic mutations); and other neurodegenerative diseases involving microglial and astrocytic inflammatory responses prior to being administered an effective amount of cPMP. In some instances, an individual may be identified as having a mutation in the MOCS1 or MOCS2 gene or the gene encoding gephyrin prior to being administered an effective amount of cPMP. cPMP can be administered on a long-term basis (e.g., when genetic mutations are present) or cPMP can be administered as an acute intervention to renormalize inhibitory synapses.

Also as described herein, treating a neurological inflammatory disease can further include monitoring the individual. Simply by way of example, the amount of MPT, MoCo, MoCo-S or another intermediate or by-product of the MoCo biosynthesis pathway (e.g., levels of xanthine, hypoxanthine, uric acid, sulfite, and S-sulfocysteine) can be monitored in an individual (e.g., in urine) and can be used as biomarkers for effective cPMP dosing. In some instances, the individual's symptoms can be monitored (e.g., for improvement) or feedback from EEG can be used to monitor treatment and/or establish dosing. Depending upon the results of the monitoring step, the effective amount of cPMP can be adjusted as desired.

Typically, an effective amount of cPMP is an amount that treats (e.g., ameliorates, relieves or reduces the symptoms of) a neurological inflammatory disease without inducing any adverse effects. An effective amount of cPMP can be formulated, along with a pharmaceutically acceptable carrier, for administration to an individual. The particular formulation, will be dependent upon a variety of factors, including route of administration, dosage and dosage interval of a compound the sex, age, and weight of the individual being treated, the severity of the affliction, and the judgment of the individual's physician. As used herein, "pharmaceutically acceptable carrier" is intended to include any and all excipients, solvents, dispersion media, coatings, antibacterial and anti-fungal agents, isotonic and absorption delaying agents, and the like, compatible with administration. The use of such media and agents for pharmaceutically acceptable carriers is well known in the art. Except insofar as any conventional media or agent is incompatible with a compound, use thereof is contemplated.

Pharmaceutically acceptable carriers are well known in the art. See, for example Remington: The Science and Practice of Pharmacy, University of the Sciences in Philadelphia, Ed., 21st Edition, 2005, Lippincott Williams & Wilkins; and The Pharmacological Basis of Therapeutics, Goodman and Gilman, Eds., 12th Ed., 2001, McGraw-Hill Co. Pharmaceutically acceptable carriers are available in the art, and include those listed in various pharmacopoeias. See, for example, the U.S. Pharmacopeia (USP), Japanese Pharmacopoeia (JP), European Pharmacopoeia (EP), and British pharmacopeia (BP); the U.S. Food and Drug Administration (FDA) Center for Drug Evaluation and Research (CDER) publications (e.g., Inactive Ingredient Guide (1996)); and Ash and Ash, Eds. (2002) Handbook of Pharmaceutical Additives, Synapse Information Resources, Inc., Endicott, NY The type of pharmaceutically acceptable carrier used in a particular formulation can depend on various factors, such as, for example, the physical and chemical properties of cPMP, the route of administration, and the manufacturing procedure.

A pharmaceutical composition that includes cPMP as described herein typically is formulated to be compatible with its intended route of administration. Suitable routes of administration include, for example, oral, rectal, topical, nasal, pulmonary, ocular, intestinal, and parenteral administration. Routes for parenteral administration include intravenous, intramuscular, and subcutaneous administration, as well as intraperitoneal, intra-arterial, intra-articular, intracardiac, intracisternal, intradermal, intralesional, intraocular, intrapleural, intrathecal, intrauterine, and intraventricular administration.

For intravenous injection, for example, the composition may be formulated as an aqueous solution using physiologically compatible buffers, including, for example, phosphate, histidine, or citrate for adjustment of the formulation pH, and a tonicity agent, such as, for example, sodium chloride or dextrose. For oral administration, a compound can be formulated in liquid or solid dosage forms, and also formulation as an instant release or controlled / sustained release formulations. Suitable dosage forms for oral ingestion by an individual include tablets, pills, hard and soft shell capsules, liquids, gels, syrups, slurries, suspensions, and emulsions.

Oral dosage forms can include excipients; excipients include, for example, fillers, disintegrants, binders (dry and wet), dissolution retardants, lubricants, glidants, anti-adherants, cationic exchange resins, wetting agents, antioxidants, preservatives, coloring, and flavoring agents. Specific examples of excipients include, without limitation, cellulose derivatives, citric acid, dicalcium phosphate, gelatine, magnesium carbonate, magnesium / sodium lauryl sulfate, mannitol, polyethylene glycol, polyvinyl pyrrolidone, silicates, silicium dioxide, sodium benzoate, sorbitol, starches, stearic acid or a salt thereof, sugars (e.g., dextrose, sucrose, lactose), talc, tragacanth mucilage, vegetable oils (hydrogenated), and waxes.

cPMP as described herein also can be formulated for parenteral administration (e.g., by injection). Such formulations are usually sterile and, can be provided in unit dosage forms, e.g., in ampoules, syringes, injection pens, or in multi-dose containers, the latter usually containing a preservative. The formulations may take such forms as suspensions, solutions, or emulsions in oily or aqueous vehicles, and may contain other agents, such as buffers, tonicity agents, viscosity enhancing agents, surfactants, suspending and dispersing agents, antioxidants, biocompatible polymers, chelating agents, and preservatives. Depending on the injection site, the vehicle may contain water, a synthetic or vegetable oil, and/or organic co-solvents. In certain instances, such as with a lyophilized product or a concentrate, the parenteral formulation would be reconstituted or diluted prior to administration. Polymers such as poly(lactic acid), poly(glycolic acid), or copolymers thereof, can serve as controlled or sustained release matrices, in addition to others well known in the art.

In accordance with the present invention, there may be employed conventional molecular biology, microbiology, biochemical, and recombinant DNA techniques within the skill of the art. Such techniques are explained fully in the literature. The invention will be further described in the following examples.

### EXAMPLES

### Example 1-Effects of IFN gamma on Neurons

Mouse cortical neurons were cultured in a two-chamber device that separates cell bodies from axons. A schematic of the chambered device constructed in PDMS polymer is shown in Figure 4A.

The experimental design is shown in Figure 4F. IFN gamma was added to the pure axons in the axon chamber, and RNA was collected from the cell body chamber 72 hours later. The RNA was analyzed by microarray to identify changes in gene expression.

A low-magnification image of the regions shown in Panel A designated "C," "D" and "E" are shown in Figure 4B, and were stained with an antibody against neurofilament, an axon-specific protein. Higher magnification images were obtained of the cell body chamber (Figure 4C), of the axon grooves (Figure 4D), and of the axon chamber (Figure 4E). DAPI staining indicated the complete absence of any cells in the axon chamber.

The results of these experiments demonstrated IFN gamma-induced dysregulation of the MoCo pathway and down-regulation of inhibitory synaptic proteins.

### Example 2-Genes Regulated by IFN gamma

IFN gamma stimulation of the distal axons stimulated a transcriptional program in the neuron cell bodies that is marked by simultaneous down-regulation of numerous components of inhibitory synapses including, for example, gephyrin (Figure 5A), glycine receptor beta subunit (Figure 5B), numerous GABA receptor elements (not shown), and multiple gephyrin-binding scaffolds (not shown) as well as robust up-regulation of MOCOS (Figure 5C). At the same time, GTP cyclohydrolase I (Figure 5D), xanthine dehydrogenase (Figure 5E), and aldehyde dehydrogenase (Figure 5F) are significantly up-regulated. Taken together, these results indicate substantial changes in the MoCo pathway.

### Example 3-Pathogenic Model

While it is known that inflammatory cytokines alter neuron excitability, the underlying mechanism by which this occurs is poorly understood. Some evidence indicates that cytokines such as TNF alpha induce changes in the distribution of excitatory and inhibitory receptors on the plasma membrane of synapses, resulting in an overall alteration in excitability (Stellwagen et al., 2005, J. Neurosci., 25:3219-28). And while the impact of inflammatory cytokines on synaptic function has been widely reviewed (see, e.g., Fouregeaud and Boulanger, 2010, Eur. J. Neurosci., 32:207-17; Koller et al., 1997, Prog. Neurobiol., 52: 1-26; Schafers and Sorkin, 2008, Neurosci. Lett., 437: 188-93), the field stills lacks a therapeutically tractable pathogenic model for the described phenomenon.

It is proposed herein that a key mechanism of hyperexcitability and seizure induction by inflammatory cytokines is the destabilization of the homeostatic molybdenum cofactor biosynthesis pathway via a reduction in gephyrin-mediated transition from MPT to MoCo, a disruption of gephyrin-mediated inhibitory neurotransmitter receptor synaptic clustering, a metabolic switch from energy production via xanthine dehydrogenase to energy failure via xanthine oxidase activity, the reversal of cPMP production from GTP to the production of 7,8-DHNP-3'-TP with concomitant amplification of nitric oxide production, the stress-dependent down-regulation of multiple components of the inhibitory neurotransmitter receptor machine, and the compensatory up-regulation of elements of the molybdenum biosynthetic apparatus. This is a novel hypothesis that places molybdenum cofactor synthesis, and particularly cPMP, at the center of a pathogenic cascade that results in severe clinical sequelae for many children. Figures 2 and 3 outline variations of this proposed model.

### Example 4-Expression Data

Cortical neurons were prepared from embryonic day 15 C57BL/6 mouse fetuses, following published protocols (Sauer et al., 2013, Neurobiol. Dis., 59:194-205). In preliminary experiments, after one week in vitro, the neurons were stimulated for 24 hr with IFN gamma (500 U/mL). Quadruplicate RNA samples were collected under treated and untreated conditions, and changes in gene expression were assessed using the Illumina BeadArray system. Only genes that were detected at P<0.05 on the array were considered for further analysis. Expression levels were un-normalized and the relative level of expression following IFN gamma stimulation was compared to untreated controls. Table 1 provides mean ± 95% confidence intervals; the appropriate statistical test was chosen based on normality and equal variance tests.

**Table 1. IFN gamma-induced Transcriptional Changes in Mouse Cortical Neurons**

| Gene | Role | Fold Change | Statistics |
|---|---|---|---|
| GABA A R α3 | inhibitory neurotransmitter receptor subunit | 0.66 ± 0.10 | P=0.0009 |
| GABA A R β1 | inhibitory neurotransmitter receptor subunit | 0.56 ± 0.09 | P=0.0001 |
| GABA A R β2 | inhibitory neurotransmitter receptor subunit | 0.80 ± 0.09 | P=0.03 |
| GABA A R β3 | inhibitory neurotransmitter receptor subunit | 0.75 ± 0.07 | P=0.0009 |
| GABA A R γ2 | inhibitory neurotransmitter receptor subunit | 0.64 ± 0.05 | P=0.007 |
| GABA B R2 | inhibitory neurotransmitter receptor subunit | 0.71 ± 0.10 | P=0.004 |
| Glycine R β1 | inhibitory neurotransmitter receptor subunit | 0.59 ± 0.07 | P=0.0001 |
| Gephyrin | GABA and glycine receptor scaffold molybdenum cofactor synthesis catalyst | 0.75 ± 0.05 | P<0.0001 |
| Collybistin | gephyrin clustering regulator | 0.71 ± 0.06 | P=0.0006 |
| MOCOS | molybdenum cofactor sulfurase | 2.97 ± 0.21 | P<0.0001 |
| Xdh | xanthine dehydrogenase; energy metabolism | 1.47 ± 0.14 | P=0.0001 |
| Aldh1a1 | aldehyde dehydrogenase; metabolism | 2.46 ± 0.08 | P<0.0001 |
| Gch1 | GTP cyclohydrolase I; biopterin synthesis | 1.59 ± 0.04 | P=0.003 |
| Actb | actin; housekeeping | 1.04 ± 0.06 | P=0.407 |
| Gapdh | glyceraldehyde 3-phosphate dehydrogenase | 1.03 ± 0.06 | P=0.850 |

In order to determine the relevance of these genes to acute inflammatory events in vivo, C57BL/6 mice were infected with the Theiler's murine encephalomyelitis virus, as per standard protocols (Howe et al., 2012a, J. Neuroinflamm., 9:50; Howe et al., 2012b, Sci. Rep., 2:545; Lafrance-Corey and Howe, 2011, J. Vis. Exp., 52:2747). The hippocampus was excised at 24 hr after infection, RNA was collected, and Illumina BeadArray analysis was performed to compare gene expression levels to sham infected mice. This time point coincided with a robust inflammatory infiltrate present in the hippocampus, and it was shown that this infiltrate triggers hippocampal neural circuitry changes that result in seizures between 3 and 7 days after infection, followed by the development of epilepsy after 60 days post-infection. It was found that GTP cyclohydrolase I was up-regulated more than 4-fold in the TMEV infected mice; likewise, MOCOS was up-regulated over 3-fold, Xdh was increased by 5-fold, several aldehyde dehydrogenase isoforms were up-regulated, as was collybistin. In contrast, the delta subunit of the GABA A receptor was down-regulated by 30%, and numerous GABA receptor subunits and binding proteins were down-regulated by 10%.

### Example 5-The Impact of Inflammatory Cytokines on Molybdenum Co-Factor Biosynthesis Gene Expression and Inhibitory Neurotransmitter Receptor Function Gene Expression

Cortical and hippocampal neurons are prepared from C57BL/6 mice and are cultured under conditions that promote formation of mature synaptic networks. Cultures are exposed to TNFalpha, IL-1β, IL-6, and IFNgamma at several doses (0, 1, 3, 10, 30, 100, 300 ng/mL) and for different times (6, 12, 24, 48, 72, and 96 hr). In parallel cultures, the amount of cell death is assessed using the MTT assay, and doses that kill greater than 10% of the culture are excluded from analysis. RNA is collected using Qiagen RNeasy kits and cDNAs are generated using the Roche Transcriptor first strand cDNA synthesis kit and random hexamer primers. Probe-based real-time PCR is performed on the samples using the Roche LightCycler 480 Probes Master system, and the primer pairs and Roche Universal Probe Library hydrolysis probes defined in Table 2. Expression is normalized to Aco2 and UROD, genes that previously have been defined as suitable housekeeping factors. A multi-factor normalization scheme is used to quantify relative differences in gene expression between controls and cytokine treated samples (Anderson et al., 2004, Cancer Res., 64:5245-50).

**Table 2. RT-PCR Analysis of Inflammatory Cytokine-Induced Changes in MOCO Biosynthesis Pathway and Inhibitory/Excitatory Receptor Expression.**

| **Gene Target** | **FORWARD** | **SEQ ID NO** | **REVERSE** | **SEQ ID NO** | **UPL PROBE** |
|---|---|---|---|---|---|
| Gephyrin var 1 (NM_145965) | tggtctcatcagttattcccatc | 1 | cgagaaatgatggagtctgga | 2 | 96 |
| Gephyrin var 2 (NM_172952) | tgatcttcatgctcagatcca | 3 | gcaaatgttgttggcaagc | 4 | 53 |
| Collybistin var 1 (NM_001033329) | tgaataaaaaggcaacctaccg | 5 | tgggagatgtcaatctctgttc | 6 | 83 |
| Collybistin var 2 (NM_001290384) | tgagaaaagcttctaaacagaaagg | 7 | gtactggccctggtttaacg | 8 | 77 |
| Mocos (NM_026779) | aagcaaagtccacacttccag | 9 | gtagtaccgggaggctgacc | 10 | 72 |
| Mocs1 (NM_020042) | ggatgtggtggacatcgtg | 11 | gaggccgatggttctcag | 12 | 11 |
| Mocs2 (NM_013826) | tattccatcggcttggtttg | 13 | tgtgagctgaagacacagca | 14 | 47 |
| Mocs3 (NM_001160330) [non-intron spanning] | ggccagatgaccgtctacc | 15 | gccgtctgcacagttggt | 16 | 94 |
| Suox (NM_173733) | tctaccatgagcatcggtgt | 17 | catcgaagacctcagagccta | 18 | 109 |
| Xdh (NM_011723) | agggattccggacctttg | 19 | gcagcagtttgggttgtttc | 20 | 69 |
| Aldh1a1 (NM_013467) | gccatcactgtgtcatctgc | 21 | catcttgaatccaccgaagg | 22 | 26 |
| Aldh1a2 (NM_009022) | catggtatcctccgcaatg | 23 | gcgcatttaaggcattgtaac | 24 | 33 |
| Aldh1a3 (NM_053080) | aacctggacaaagcactgaag | 25 | aatgcattgtagcagttgatcc | 26 | 66 |
| Aldh1a7 (NM_011921) | caagctggctgacttaatgga | 27 | gactttcccagcattcatcg | 28 | 84 |
| Aldh1b1 (NM_028270) | gaccggagaacgctgatacta | 29 | agtcgggcagtcagcatc | 30 | 45 |
| Aldh1l1 (NM_027406) | tccctacttcccgtctttga | 31 | acaggctctgcccgattac | 32 | 97 |
| Aldh1l2 (NM_153543) | cgctcgctcctctacatcat | 33 | gccaacttcagcttgtttttg | 34 | 22 |
| Aldh2 (NM_009656) | tgttcggggacgtaaaagac | 35 | tgaggatttgcatcactggt | 36 | 63 |
| Aldh3a1 (NM_007436) | tgtggagaagctcaaaaagtcac | 37 | cccatagtcatgggactgct | 38 | 60 |
| Aldh3a2 (NM_007437) | gctgaagcagttcaacaaagg | 39 | aggagaggcaacaaggaagtc | 40 | 89 |
| Aldh3b1 (NM_026316) | ctggatgaagccatcgagtt | 41 | ccaacacctgtttgataacctg | 42 | 7 |
| Aldh3b2 (NM_001177438) | aagagttgctgcacatctgg | 43 | gtgcagtgtggcctcaga | 44 | 84 |
| Aldh4a1 (NM_175438) | accggttccgtaccttcc | 45 | agctgtgcacaaaatggaaat | 46 | 19 |
| Aldh5a1 (NM_172532) | aacagctggaaaggggtctc | 47 | cattaagtcgtaccatttacggagt | 48 | 55 |
| Aldh6a1 (NM_134042) | ggtaaatgccacatggtatcc | 49 | tgattcaacaaattttccatcaat | 50 | 88 |
| Aldh7a1 (NM_138600) | tgtccttcactgggagcact | 51 | gtttcctccaagctccaaca | 52 | 10 |
| Aldh8a1 (NM_178713) | ggcaagaatcctgctatcatct | 53 | actggtacaaaggcaaatctcc | 54 | 25 |
| Aldh9a1 (NM_019993) | ctggcagtgcctggaatact | 55 | cctcctgggagctgaatgt | 56 | 71 |
| Aldh16a1 (NM_145954) | ctcttgggccaccatgtc | 57 | ctggccaggttttctcctg | 58 | 5 |
| Aldh18a1 (NM_019698) | gatgtcatcgtcacagagaacg | 59 | aagacacaggcgctgtcc | 60 | 67 |
| GTP Cyclohydrolase 1 Gch1 (NM_008102) | gcctcaccaaacagattgc | 61 | cacgcctcgcattaccat | 62 | 40 |
| Nitric Oxide Synthase 1 (neuronal) Nos1 (NM_008712) | ggcgttcgtgattactgtga | 63 | tcttcctcatgtccaaatcca | 64 | 69 |
| Nitric Oxide Synthase 2 (inducible) Nos2 (NM_010927) | ctttgccacggacgagac | 65 | tcattgtactctgagggctgac | 66 | 13 |
| Glycine Receptor Glra1 (NM_001290821) | caacacaaggaactgcttcg | 67 | gatttagcatggggctcttg | 68 | 69 |
| Glycine Receptor Glra2 (NM_183427) | cataaggagttccttcgtctc | 69 | cgactttcacgagtaacatcttctt | 70 | 34 |
| Glycine Receptor Glra3 (NM_080438) | gggaagccgcactgttact | 71 | gagatcgcgcactgtttgt | 72 | 49 |
| Glycine Receptor Glrb1 (NM_010298) | ctgatgctagtgctgccaga | 73 | gtgcactctgaggccaaact | 74 | 105 |
| GABA A Receptor Gabra1 (NM_010250) | gcccactaaaattcggaagc | 75 | cttctgctacaaccactgaacg | 76 | 93 |
| GABA A Receptor alpha Gabra2 (NM_008066) | acaaaaagaggatgggcttg | 77 | tcatgacggagcctttctct | 78 | 103 |
| GABA A Receptor alpha Gabra3 (NM_008067) | cttgggaaggcaagaaggta | 79 | tggagctgctggtgttttct | 80 | 80 |
| GABA A Receptor alpha Gabra4 (NM_010251) | aaagcctcccccagaagtt | 81 | catgttcaaattggcatgtgt | 82 | 68 |
| GABA A Receptor alpha Gabra6 (NM_001099641) | caagtgtctttctggattaataaggag | 83 | gtcatggttaaaacagtggtgatt | 84 | 95 |
| GABA A Receptor beta Gabrb1 (NM_008069) | ccctctggatgagcaaaact | 85 | aattcgatgtcatccgtggta | 86 | 80 |
| GABA A Receptor beta Gabrb2 (NM_008070) | gggtctccttttggattaactatga | 87 | ggtcattgttaggacagttgtaattc | 88 | 84 |
| _ GABA A Receptor beta Gabrb3 (NM_008071) | ctccattgtagagcaccgtct | 89 | tcaatgaaagtcgaggataggc | 90 | 80 |
| GABA A Receptor delta Gabrd (NM_008072) | cggagctgatgaacttcaaat | 91 | atgtagacgccccggttc | 92 | 11 |
| GABA A Receptor gamma 2 Gabrg2 (NM_008073) | acagaaaatgacgctgtgga | 93 | catctgacttttggcttgtgaa | 94 | 68 |
| GABA A Receptor gamma 3 Gabrg3 (NM_008074) | atgcgacaccagcaagaac | 95 | caatggtgctgagtgtggtc | 96 | 9 |
| GABA A Receptor pi Gabrp (NM_146017) | atttcaccctggtcaccgta | 97 | gctcaaattgcaaaaccaatc | 98 | 18 |
| GABA A Receptor rho Gabrr1 (NM_008075) | tgcctgctagagtcccctta | 99 | ccgtgatgatggtggacat | 100 | 9 |
| GABA B Receptor 1 Gabbr1 (NM_019439) | gacattgatgtctccattctgc | 101 | gcagccctttgtaaccataga | 102 | 78 |
| GABA B Receptor 2 Gabbr2 (NM_001081141) | gaacatggcagcgaaagtct | 103 | ctggtacttgctgccaaaca | 104 | 91 |
| Glutamate Receptor AMPA 1 Gria1 (NM_008165) | agggatcgacatccagagag | 105 | tgcacatttcctgtcaaacc | 106 | 62 |
| Glutamate Receptor AMPA 2 Gria2 (NM_013540) | ggggaggtgattccaagg | 107 | cccccgacaaggatgtaga | 108 | 67 |
| Glutamate Receptor AMPA 3 Gria3 (NM_016886) | agccgtgtgatacgatgaaa | 109 | caaggtttacaggcgttcct | 110 | 31 |
| Glutamate Receptor AMPA 4 Gria4 (NM_019691) | ctgccaacagttttgctgtg | 111 | aaatggcaaacacccctcta | 112 | 48 |
| Glutamate Receptor Delta 1 Grid1 (NM_008166) | agacttttggcaaagacatgc | 113 | actgccattcaagcccttc | 114 | 95 |
| Glutamate Receptor Delta 2 Grid2 (NM_008167) | ccctaccgtgatgtcttttca | 115 | agaatgtccatgtcgccact | 116 | 1 |
| Glutamate Receptor Kainate 1 Grik1 (NM_146072) | tctggtttggcgttggag | 117 | tcctccaactattctggtcgat | 118 | 105 |
| Glutamate Receptor Kainate 2 Grik2 (NM_010349) | agtgccaccataccatccag | 119 | gctggcacttcagagacattc | 120 | 31 |
| Glutamate Receptor Kainate 3 Grik3 (NM_001081097) | cacttcatcttcaccactctgg | 121 | actcccgagtagcggtagg | 122 | 85 |
| Glutamate Receptor Kainate 4 Grik4 (NM_175481) | gccattgagtatggcacgat | 123 | tggtaacgggaattttggaa | 124 | 67 |
| Glutamate Receptor Kainate 5 Grik5 (NM_008168) | cccctcagctagcctcatct | 125 | gcctcgcaccagttcttcta | 126 | 40 |
| Glutamate Receptor NMDA 1 Grin1 (NM_008169) | catttagggctatcacctcca | 127 | cactgtgtctttttggttttgc | 128 | 78 |
| Glutamate Receptor NMDA 2A Grin2a (NM_008170) | attcaaccagaggggcgta | 129 | ttcaagacagctgcgtcatag | 130 | 48 |
| Glutamate Receptor NMDA 2B Grin2b (NM_008171) | gggttacaaccggtgccta | 131 | ctttgccgatggtgaaagat | 132 | 53 |
| Glutamate Receptor NMDA 2C Grin2c (NM_010350) | gaagcgggccatagacct | 133 | tggcagatccctgagagc | 134 | 94 |
| Glutamate Receptor NMDA 2D Grin2d (NM_008172) | tgcgatacaaccagccaag | 135 | agatgaaggcgtccagtttc | 136 | 25 |
| Glutamate Receptor NMDA 3A Grin3a (NM_001276355) | cacgaatcaaaaacaaatccaa | 137 | tgtgtttaatgctctgtgaaacc | 138 | 71 |
| Glutamate Receptor NMDA 3B Grin3b (NM_130455) | cgtcctacggagggaggt | 139 | ccagggacaccagcacat | 140 | 80 |
| Glutamate Receptor NMDA AP1 Grina (NM_023168) | ctcaaggaggctacccacag | 141 | tagttcccatgctgaggtga | 142 | 77 |
| PSD-95 Dlg4 (NM_007864) | tctgtgcgagaggtagcaga | 143 | cggatgaagatggcgatag | 144 | 102 |

### Example 6-The Impact of Inflammatory Cytokines on Molybdenum Co-Factor Biosynthesis and Inhibitory Neurotransmitter Receptor Function Protein Expression

Similar cultures and treatment conditions as described in Example 5 are used to generate protein lysates for analysis of expression of GABA receptor subunits, glycine receptor subunits, gephyrin, GTP cyclohydrolase I, and MoCoS. Neurons grown in glass multi-well chambered slides are used for the analysis of expression of these targets by immunofluorescence microscopy. For IF, cells are stimulated for 24, 48, 72, or 96 hrs at 100 ng/mL (or at a dose defined in Example 5 as optimal for gene induction) prior to fixation and immunostaining. Table 3 lists the relevant antibodies that are employed.

**Table 3. Antibodies**

| Target | Source |
|---|---|
| GABA A R α1 | Synaptic Sys #224203, 1:500 (WB), rabbit |
| | Alomone #AGA-001, 1:400 (IF), rabbit |
| GABA A R α2 | Synaptic Sys #224102, 1:500 (WB), rabbit |
| | Alomone #AGA-002, 1:200 (IF), rabbit |
| GABA A R α3 | Synaptic Sys #224302, 1:500 (WB), rabbit |
| | Alomone #AGA-003, 1:200 (IF), rabbit |
| GABA A R β2/3 | Millipore #MAB341, 1:50 (WB), 1:25 (IF), mouse |
| GABA A R γ2 | Alomone #AGA-005, 1:200 (WB), 1:100 (IF), rabbit |
| GABA B R2 | Synaptic Sys #322205, 1:1000 (WB), 1:500 (IF), guinea pig |
| Glycine R β | Alomone #AGR-014, 1:200 (WB), rabbit |
| | Synaptic Sys #146211, 1:500 (IF), mouse |
| Gephyrin | Synaptic Sys #147004, 1:1000 (WB), guinea pig |
| | Synaptic Sys #147021, 1:250 (IF), mouse |
| Collybistin | Synaptic Sys #261003, 1:1000 (WB), 1:500 (IF), rabbit |
| GTP Cyclohydrolase I | Sigma #SAB4200046-200UL, 1:1000 (WB) |
| MoCo-S | Santa Cruz #SC-85066, 1:200 (WB), rabbit |
| | Pierce #PA5-30662, 1:500 (WB), rabbit |

### Example 7-The Impact of Inflammatory Cytokines on Spontaneous and Evoked Synaptic Activity

Neurons are cultured in glass imaging chambers under conditions that promote formation of mature synaptic networks. Cells are infected with an AAV1.Syn.GCaMP6f calcium reporter that provides fast optical tracking of intracellular calcium levels (Akerboom et al., 2012, J. Neurosci., 32:13819040; Chen et al., 2013, Nature, 499:295-300). Calcium levels are monitored in real-time using a Zeiss 5-Live confocal microscope equipped with environmental chamber. Following collection of baseline spontaneous activity levels at low magnification, inflammatory cytokine is added at the optimal cytokine concentration determined above, and cells are followed for up to 60 minutes. Images are post-processed in Image J to measure calcium transient amplitudes and frequencies within defined cells. In some experiments, an Olympus multi-photon microscope is used at high magnification to track activity in individual synapses. In addition to spontaneous activity, calcium flux elicited by addition of glutamate to cultures that have been pretreated with inflammatory cytokines for different times (0, 1, 3, 6, 12, 24, 48, 72, or 96 hr) prior to stimulation also is measured. See Figures 6 and 7.

### Example 8-The Effect of cPMP Supplementation on Synaptic Changes Induced by Inflammatory Cytokines

Hippocampal and cortical neurons are treated with inflammatory cytokines at the optimized dose and time identified above in the presence of different concentrations of cPMP. Extrapolating from the field of purinergic signaling, concentrations ranging from nanomolar to millimolar (1, 3, 10, 30, 100, 300 nM; 1, 3, 10, 30, 100, 300 µM; 1, 3 mM) are tested. In preliminary experiments, the survival of naive neurons treated with cPMP for different times (1, 3, 6, 12, 24, 48, 72, or 96 hr) is assessed by MTT or LDH assay, and doses that kill more than 10% of cells are excluded from further analysis. In some instances, the cPMP is encapsulated in liposomes (for example, lipofectin or lipofectamine) (Hughes et al., 2010, Methods Mol. Biol., 605:445-59). After optimizing cPMP delivery, neurons are stimulated with inflammatory cytokines in the presence or absence of cPMP under conditions that alter spontaneous and/or evoked calcium flux. If cPMP treatment reverses the effect of inflammatory cytokines on dynamic synaptic activity, the effect of cPMP on expression and localization of the protein targets explored in Example 6 also is examined, and the effect of cPMP on the expression of genes measured in Example 5 is tested.

### Example 9-MoCo Pathway and Neurotransmitter Receptor Changes Induced by Acute Virus Infection of the Brain

Young (4 week old) mice were infected with the Theiler's murine encephalomyelitis virus for 24 hr to model acute childhood brain infection. Illumina microarray was employed to assess transcriptional changes. Table 4 shows maximal up-regulation or down-regulation of relevant genes during the first 24 hr of infection.

**Table 4. Maximal Up-regulation or Down-regulation of Relevant Genes**

| Gene | Fold-change | Function | | Gene | Fold-change | Function |
|---|---|---|---|---|---|---|
| MoCos | 3.0 | molybdenum cofactor biosynthesis | | Gphn | -1.4 | gephyrin (Mo pathway and inhibitory synapse) |
| Xdh | 4.9 | xanthine dehydrogenase/oxidase | | Glra2 | -1.4 | glycine receptor (inhibitory) |
| Gch 1 | 4.3 | GTP cyclohydrolase 1 | | Gabrd | -1.6 | GABA receptor (inhibitory) |
| Aldh1b1 | 2.5 | aldehyde dehydrogenase 1 | | Gabrb1 | -1.4 | GABA receptor (inhibitory) |
| Nos3 | 1.3 | nitric oxide synthase 3 | | Gabrg1 | -1.3 | GABA receptor (inhibitory) |
| Gria2 | 1.3 | AMPA receptor (excitatory) | | Gabbr2 | -1.3 | GABA receptor (inhibitory) |
| Grin2c | 1.2 | NMDA receptor (excitatory) | | Gabbr3 | -1.3 | GABA receptor (inhibitory) |
| Dlg5 | 1.3 | disks large homolog (excitatory) | | Gabbr1 | -1.3 | GABA receptor (inhibitory) |
| Capn2 | 1.5 | large calpain subunit | | Gabra1 | -1.3 | GABA receptor (inhibitory) |
| Capn5 | 1.3 | large calpain subunit | | Gad 1 | -1.3 | glutamate decarboxylase (inhibitory) |
| Capns1 | 1.3 | small calpain subunit | | | | |
| Gpx1 | 1.5 | glutathione peroxidase 1 | | | | |
| Akr1b8 | 1.8 | aldo/keto reductase 1 | | | | |
| G6pdx | 1.3 | glucose-6-P dehydrogenase | | | | |

These measurements indicate that acute infection of the brain, consistent with elevated TNF alpha (6-fold increase at 24 hr) and IL1 beta (10-fold increase at 24 hr) in this model system, induces increased synthesis of MoCo pathway-related factors, increased production of oxidative stress factors, up-regulated calpain production, and increased expression of excitatory neurotransmitter receptors. Simultaneously, acute infection triggers down-regulated expression of gephyrin and a host of GABAergic receptors, resulting in suppression of synaptic inhibition.

### Example 10-MoCo Pathway and Neurotransmitter Receptor Changes in Human Neurons Induced by Inflammatory Cytokines

Neurons were induced from human neural stem cells and grown under conditions that foster mixed excitatory and inhibitory neuron phenotypes. These cells were then stimulated with TNF alpha, IL1 beta, or IFN gamma for 24 hrs, and transcriptional changes were assessed by microarray. Responses were variable between cytokines but, in general, the inflammatory stimuli induced changes that are summarized in Table 5.

**Table 5. Inflammatory Stimuli Induced Genes**

| Gene | Fold-change | Function | | Gene | Fold-change | Function |
|---|---|---|---|---|---|---|
| MoCos | 2.0 | molybdenum cofactor biosynthesis | | Gphn | -1.2 | gephyrin (Mo pathway and inhibitory synapse) |
| Gch1 | 3.7 | GTP cyclohydrolase 1 | | Glra2 | -1.7 | glycine receptor (inhibitory) |
| Aldh1 | 1.5 | aldehyde dehydrogenase 1 | | Glrb | -1.7 | glycine receptor (inhibitory) |
| Aldh2 | 1.4 | aldehyde dehydrogenase 2 | | Gabrd | -1.6 | GABA receptor (inhibitory) |
| Nos3 | 1.6 | nitric oxide synthase 3 | | Gabbr2 | -5.0 | GABA receptor (inhibitory) |
| Gria3 | 1.4 | AMPA receptor (excitatory) | | Gabbr3 | -2.5 | GABA receptor (inhibitory) |
| Grina | 1.4 | NMDA receptor (excitatory) | | Gad1 | -1.8 | glutamate decarboxylase (inhibitory) |
| Grin3a | 2.3 | NMDA receptor (excitatory) | | | | |
| Grik2 | 1.5 | | | | | |
| Grm5 | 3.9 | metabotropic glutamate receptor (excitatory) | | | | |
| Dlg3 | 1.2 | disks large homolog (excitatory) | | | | |
| Capn2 | 1.5 | large calpain subunit | | | | |
| Capn5 | 5.3 | large calpain subunit | | | | |
| Capns1 | 1.2 | small calpain subunit | | | | |

### Example 11-MoCo Pathway and Neurotransmitter Receptor Changes in Human Neurons Induced by Inflammatory Cytokines

Neurons were cultured from neonatal mice and stimulated with TNF alpha or IFN gamma for 24 hr. Transcriptional changes were assessed by quantitative RT-PCR.

**Table 6. Transcriptional Changes**

| Gene | Fold-change | Function | | Gene | Fold-change | Function |
|---|---|---|---|---|---|---|
| Gria1 | 1.2 | AMPA receptor (excitatory) | | Gphn | -2.1 | gephyrin (Mo pathway and inhibitory synapse) |
| Gria2 | 1.7 | AMPA receptor (excitatory) | | Glra2 | -1.6 | glycine receptor (inhibitory) |
| Gria3 | 4.1 | AMPA receptor (excitatory) | | Glra3 | -2.3 | glycine receptor (inhibitory) |
| Grid1 | 1.3 | glutamate receptor (excitatory) | | Glrb1 | -4.5 | glycine receptor (inhibitory) |
| Grid2 | 2.4 | glutamate receptor (excitatory) | | Gabra1 | -1.2 | GABA receptor (inhibitory) |
| Grik3 | 6.2 | kainate receptor (excitatory) | | Gabra2 | -2.3 | GABA receptor (inhibitory) |
| Grik4 | 3.0 | kainate receptor (excitatory) | | Gabra4 | -2.9 | GABA receptor (inhibitory) |
| Grik5 | 1.8 | kainate receptor (excitatory) | | Gabrb 1 | -1.4 | GABA receptor (inhibitory) |
| Grin2b | 2.6 | NMDA receptor (excitatory) | | Gabrd | -1.6 | GABA receptor (inhibitory) |
| Grin2d | 1.9 | NMDA receptor (excitatory) | | Gabrg2 | -4.8 | GABA receptor (inhibitory) |
| Grin3a | 2.7 | NMDA receptor (excitatory) | | | | |
| Grina | 2.6 | NMDA receptor (excitatory) | | | | |

### Example 12-Inflammatory Cytokines Induce Network Synchronization That Phenocopies Loss of Inhibition

Neurons were cultured from neonatal mice and stimulated with TNF alpha (100 ng/mL) or IFN gamma (500 U/mL) for 24 hr. Following transduction with an AAV-encoded GCaMPf reporter, fast calcium transients were imaged in the cells. Regions of interest outlining individual neurons were defined in each frame of movies collected over several minutes, and the fluorescence intensity of each cell was graphed through time to reveal patterns in the population response. Figure 8 shows the averaged calcium response traces calculated for dozens of cytokine-stimulated neurons in each experiment and are representative of more than 4 separate experiments and more than 4 separate cell preps within each experiment.

Figure 8A shows the basal level of calcium activity in the neuron cultures. Non-synchronized calcium responses occur in the control cultures, resulting in an overall low level of synaptic activity in the network. Figures 8B and 8C show the stimulation of calcium activity in neurons following treatment with IFN gamma or TNF alpha, respectively. Figures 8B and 8C show that treatment with IFN gamma or TNF alpha results in network bursting and highly synchronized synaptic activity in which many cells in the culture flux calcium at the same time.

Figure 8D shows that treatment of control cultures with 2.4 µM picrotoxin, a small molecule inhibitor of inhibitory GABAergic channels, induces network synchrony and bursting that phenocopies the response observed in cytokine-stimulated cultures (compare with Figures 8B and 8C). Figure 8E shows that addition of 27 µM GABA to control cultures completely suppresses synaptic activity, consistent with enhanced inhibition.

These findings indicate that TNF alpha and IFN gamma induce the suppression of inhibitory neurotransmission in the neuronal network, resulting in synchronous bursting behavior. Given the transcriptional profiles measured in cytokine-stimulated neurons, this network behavior is consistent with a reduction in inhibitory neurotransmitter receptors linked to reduced gephyrin expression and alteration of the MoCo synthesis pathway.

Disclosed are methods and compositions that can be used for, can be used in conjunction with, can be used in preparation for, or are products of the disclosed methods and compositions. These and other materials are disclosed herein, and it is understood that combinations, subsets, interactions, groups, etc. of these methods and compositions are disclosed. That is, while specific reference to each various individual and collective combinations and permutations of these compositions and methods may not be explicitly disclosed, each is specifically contemplated and described herein. For example, if a particular composition of matter or a particular method is disclosed and discussed and a number of compositions or methods are discussed, each and every combination and permutation of the compositions and the methods are specifically contemplated unless specifically indicated to the contrary. Likewise, any subset or combination of these is also specifically contemplated and disclosed.

## Claims

1. A composition for use in a method of treating a neurological inflammatory disease in an individual, comprising:
identifying an individual having ALS, epilepsy, autism, or schizophrenia,
administering an effective amount of cPMP (cyclic pyranopterin monophosphate) to the individual,
thereby treating the individual.

2. The composition for use of claim 1, wherein the neurological inflammatory disease is selected from the group consisting of central nervous system (CNS) autoimmune disorders such as autoimmune epilepsies; amyotrophic lateral sclerosis (ALS); schizophrenia; autism; epilepsy; and febrile seizures without underlying infection.

3. The composition for use of claim 1, wherein the administering step is selected from the group consisting of orally administering, topically administering, and parenterally administering.

4. The composition for use of claim 1, further comprising identifying an individual having a mutation in a gene selected from the group consisting of gephyrin, MOCS1, and MOCS2.

5. The composition for use of claim 1, further comprising monitoring the individual for the amount of MPT, MoCo, MoCo-S or another intermediate or by-product of the MoCo biosynthesis pathway.

## Patentansprüche

1. Zusammensetzung zur Verwendung bei einem Verfahren zur Behandlung einer neurologischen Entzündungskrankheit bei einem Individuum, das Folgendes umfasst:
Identifizieren eines Individuums mit ALS, Epilepsie, Autismus oder Schizophrenie,
Verabreichen einer wirksamen Menge von cPMP (cyclischem Pyranopterinmonophosphat) an das Individuum,
wodurch das Individuum behandelt wird.

2. Zusammensetzung zur Verwendung nach Anspruch 1, wobei die neurologische Entzündungskrankheit aus der Gruppe bestehend aus Autoimmunstörungen des Zentralnervensystems (ZNS) wie Autoimmunepilepsien, amyotropher Lateralsklerose (ALS), Schizophrenie, Autismus, Epilepsie und Fieberkrämpfen ohne zugrundeliegende Infektion ausgewählt ist.

3. Zusammensetzung zur Verwendung nach Anspruch 1, wobei der Verabreichungsschritt aus der Gruppe bestehend aus oraler Verabreichung, tropischer Verabreichung und parenterale Verabreichung ausgewählt ist.

4. Zusammensetzung zur Verwendung nach Anspruch 1, ferner umfassend das Identifizieren eines Individuums mit einer Mutation in einem Gen, das aus der Gruppe bestehend aus Gephyrin, MOCS1 und MOCS2 ausgewählt ist.

5. Zusammensetzung zur Verwendung nach Anspruch 1, ferner umfassend das Überwachen des Individuums hinsichtlich der Menge von MPT, MoCo, MoCo-S oder einem anderen Zwischenprodukt oder Nebenprodukt des MoCo-Biosynthesewegs.

## Revendications

1. Composition pour une utilisation dans un procédé de traitement d'une maladie inflammatoire neurologique chez un individu, comprenant :
l'identification d'un individu atteint d'ALS, d'épilepsie, d'autisme, ou de schizophrénie,
l'administration d'une quantité efficace de cPMP (monophosphate de pyranoptérine cyclique) à l'individu,
traitant ainsi l'individu.

2. Composition pour une utilisation selon la revendication 1, la maladie inflammatoire neurologique étant choisie dans le groupe constitué par des troubles auto-immuns du système nerveux central (SNC) comme les épilepsies auto-immunes ; la sclérose latérale amyotrophique (SLA) ; la schizophrénie ; l'autisme ; l'épilepsie ; et les crises fébriles sans infection sous-jacente.

3. Composition pour une utilisation selon la revendication 1, l'étape d'administration étant choisie dans le groupe constitué par une administration par voie orale, une administration par voie topique, et une administration par voie parentérale.

4. Composition pour une utilisation selon la revendication 1, comprenant en outre l'identification d'un individu possédant une mutation dans un gène choisi dans le groupe constitué par la géphyrine, MOCS1 et MOCS2.

5. Composition pour une utilisation selon la revendication 1, comprenant en outre la surveillance de l'individu quant à la quantité de MPT, MoCo, MoCo-S, ou d'un autre intermédiaire ou sous-produit de la voie de la biosynthèse de MoCo.
